# EUROPEAN PATENT APPLICATION

(11) **EP 1 576 877 A1**
(43) Date of publication of application: **21.09.2005**
(21) Application number: 04290589.3
(22) Date of filing: 04.03.2004
(51) Int. Cl.: A01K 67/027, C12N 15/62, A61K 39/00

(54) **Preparation of recombinant rotavirus proteins in milk of transgenic non-human mammals**

(71) Applicant: Bioprotein Technologies, 75013 Paris (FR)
(72) Inventor: Cohen, Jean, 75014 Paris (FR); Soler, Eric, 78000 Versailles (FR); Houdebine, Louis-Marie, 78530 Buc (FR)
(74) Representative: Warcoin, Jacques

(57) **Abstract**

The present invention relates to a non-human transgenic mammal whose genome comprises: i) a first transgene comprising a mammary gland specific transcriptional control region operably linked to cDNA encoding a rotavirus protein selected from VP2, VP4, VP6 and VP7 and wherein said cDNA comprises a secretion signal sequence; ii) at least a second transgene comprising a mammary gland specific transcriptional control region operably linked to cDNA encoding another said rotavirus protein and wherein said cDNA comprises a secretion signal sequence; and wherein said rotavirus proteins are secreted separately and auto-assembled in milk in rotavirus like particles (VLP) or aggregates of said rotavirus proteins.

## Description

The present invention relates to a non-human transgenic mammal whose genome comprises: i) a first transgene comprising a mammary gland specific transcriptional control region operably linked to cDNA encoding a rotavirus protein selected from VP2, VP4, VP6 and VP7 and wherein said cDNA comprises a secretion signal sequence; ii) at least a second transgene comprising a mammary gland specific transcriptional control region operably linked to cDNA encoding another said rotavirus protein and wherein said cDNA comprises a secretion signal sequence; and wherein said rotavirus proteins are secreted separately and auto-assembled in milk in rotavirus like particles (VLP) or aggregates of said rotavirus proteins.

### Background of the invention

Rotavirus is a wide spread virus considered as democratic since it is highly contagious. Rotavirus infects children and adults, healthy or not, with an equal efficiency. Rotavirus infections are responsible for the death of 2 000 children per day and of 830 000 persons per year, most of them in developing countries. The total number of infected persons is much higher, generating transient but important troubles.

Numerous studies have demonstrated that several proteins of the rotavirus could be used as vaccine when administered by injection, or in some cases, orally. Attempts to use attenuated live vaccines were successful but accompanied by severe side-effects. A proportion of treated persons suffered from intussusception (a telescoping of a section of bowel). This vaccine was thus withdrawn. The control of this side-effect seems difficult and the use of recombinant rotavirus proteins as vaccine appears safer (Beale 2002).

More generally, the use of recombinant vaccines is considered by United Nations as the second of the top ten technique to improve health in developing countries (Acharya et al 2003). Most of the native or recombinant rotaviral proteins have been produced in small amounts and their capacity to protect experimental animals against infection has been demonstrated (Bertolotti-Ciarlet et al, 2003 ;Schwartz -Cornil et al, 2002 ; Kim et al, 2002 ; Kiang et al,1999 ; Ciarlet et al,1998 ; O'Neal et al 1997). The chances of these proteins to become efficient vaccines are not similar. Ideally, a vaccine should have an equivalent efficiency against all the major virus subtypes. Moreover, the recombinant vaccine should be produced in a relatively large amount at a low cost to be administered by injection or by oral route.

Among the rotavirus proteins to be used as vaccine, VP₂ and VP₆ appear among the best candidates. VP₆ is a capsid protein which has a well-conserved structure with more than 90% homology among group A rotaviruses. It can therefore potentially vaccinate against all the rotavirus of group A. Group A rotaviruses are mainly those infecting humans. The use of VP6 can therefore potentially vaccinate against all the members of group A independently of serotypes.
VP₂ and VP₆ spontaneously form viral like particles (VLP) which are resistant to proteases and can induce immunological protection against the virus, even when administered orally. These experiments are a proof of concept showing that VLP are quite able to vaccinate against rotavirus infections. The major limitation in the use of rotavirus VLP is their availability. Interestingly also, foreign peptides or proteins fused to VP₂ or VP₆ are integrated into the VLP and have quite significant antigenic properties (Charpilienne et al. 2001 and WO 01/66566).

Recombinant VLP can be prepared from Sf9 cells infected by baculovirus harboring VP₂ and VP₆ genes. The VLPs which can be extracted from the cell lysate share the structural and immunological properties of nascent VLPs. However, this system has limited capacity to produce recombinant proteins. The protein VP₆ has been prepared in transgenic potatoes and the tuber tissues containing the viral protein was able to induce immunity in mice after oral administration (Yu and Langridge 2003) or after injection with an adjuvant (Matsumura et al. 2002). Similarly, rotavirus protein VP₇ produced in potatoes induced a high titer of mucosal neutralizing IgA in mice (Wu and al. 2003). But, the amount of rotavirus proteins produced in potatoes was in all cases very low. Moreover, the purification of the recombinant proteins is expected to be difficult in these conditions.

It has recently been proposed in the art to use transgenic plants (Ma et al. 2003), but this system has shown limited capacity and there are no indication that plant VP proteins would be in a form suitable to assemble in VLP. In addition, the problem of the dissemination of transgenic plants containing pharmaceutical proteins has not been solved. (Ma et al. 2003).

Milk from transgenic animals is the most mature system to produce large amounts of recombinant pharmaceutical proteins (Houdebine 2000; Houdebine 2003). More than 100 of recombinant proteins have been experimentally prepared in the milk of mice, rats, rabbits, sheep, goats, pigs and cows. The first pharmaceutical protein extracted from milk, human antithrombin III, is expected to be in market in 2004. This means that the major problems, expression level, purification from milk and biosafety, have been solved.

We used our expertise in this field to design different vectors and we demonstrate, for the first time, that rotaviral proteins can be produced and secreted at a high rate in milk after the addition of a signal peptide. We fortunately observed that the proteins VP₂ and VP₆ were not aggregated to casein micelles. Thus, VP proteins can be recovered from lactoserum following low cost extraction and purification steps.

We have also discovered that recombinant VP₂ and VP₆ spontaneously form VLP or VP proteins aggregates in milk of high molecular weight further allowing purification.

### Description

Therefore, in a first aspect, the invention is aimed at a non-human transgenic mammal whose genome comprises:
i) a first transgene comprising a mammary gland specific transcriptional control region operably linked to cDNA encoding a rotavirus protein selected from VP2, VP4, VP6 and VP7 and wherein said cDNA comprises a secretion signal sequence;
ii) at least a second transgene comprising a mammary gland specific transcriptional control region operably linked to cDNA encoding another rotavirus protein selected from VP2, VP4, VP6 and VP7 and wherein said cDNA comprises a secretion signal sequence; and wherein said rotavirus protein are secreted separately and auto-assembled in milk in rotavirus like particles (VLP) or aggregates of said rotavirus proteins.

In a preferred embodiment, the first transgene comprises a cDNA encoding a VP2 rotavirus protein and the second transgene comprises a cDNA encoding a VP6 rotavirus protein. In this regards, the non-human transgenic mammal may comprise a third or fourth transgene comprising a cDNA encoding a rotavirus protein selected from VP4 and VP7.

The invention relates to the non-human transgenic mammal as defined above, wherein said VP2 and VP6 assemble in VLP or aggregates of at least 300 000 KDA. Thus, in another preferred embodiment, the invention contemplates a non-human female transgenic mammal, which milk comprise VP2, VP6, monomer and multimer thereof (for example VP6 trimers); VP2-VP6 based VLP or VP2-VP6 aggregates of at least 300 000 KDA.

In said female, the milk contains at least 10 µg/ml, preferably at least 100 µg/ml of both VP2 and VP6.

The term "genome" is intended here to include the entire endogenous DNA of a mammal, including the nuclear or extrachromosomal DNA.

It will be appreciated by those in the art that the expression "transgene" is meant to refer to nucleic acid molecule comprising a foreign sequence, which is inserted in the genome of said non-human mammal and which insertion is stable over time. The transgene is introduced into the cell by microinjection and integrates into the genome via homologous recombination or recombinase directed site specific recombination (Cre/Lox, FLP/FRT). The transgene can also be in the form of a vector which is a recombinant virus. Thus, the expression "vector" or "transgene" are interchangeably used herein. This molecule may be integrated within a chromosome, or it may be extrachromosomally replicating DNA. Classical cross-breeding, or in vitro fertilization, or direct introduction of the transgene allows the production of homozygote non-human transgenic mammals. The "transgenic non-human mammal" of the invention is preferably produced by introducing one or several "transgene" as defined above into the germline of said non-human mammal.

The mammary gland specific transcriptional control region can be selected from gene coding for a milk serum protein or a casein protein. Several milk gene promoters are used to prepare recombinant proteins in milk (see. EP 264 166 (transgenic animals secreting desired proteins into milk) and EP 527 063 (production of protein of interest in the milk of transgenic mammal).

More particularly, the mammary gland specific transcriptional control region is the WAP (whey acidic protein) promoter such as the long mouse or rabbit WAP promoter.
Examples of suitable WAP promoters are regions of at least 3 kb, 3 kb to 6.3 kb or at least 6.3 kb from the translation initiation start of the rabbit WAP promoter. A particularly advantageous rabbit long WAP promoter sequence is described in figures 1 and 5 of EP0527063 (Houdebine et al), incorporated herein by reference. The long promoter (6,3 kb) of the rabbit WAP gene (Houdebine et al. 1991) was used in the hereinafter described experiments to express VP₂ and VP₆ cDNAs. The transgene as referred herein may further comprise the genomic sequences surrounding the WAP gene, preferably at least 110Kb upstream and at least 10Kb downstream of the WAP gene from sheep, pig, goat, cow, rabbit, rat or mouse.

Experiments carried out several years ago showed that the 5'HS4 region from the chicken β-globin gene cluster dramatically enhanced the frequency of animals expressing their transgenes, with a higher expression level (Taboit-Dameron et al. 1999 ; Rival-Gervier et al. 2003). Thus, the transgene may further comprise the 5'HS4 region from the chicken β-globin gene cluster. In the following experiments, the 5'HS4 was added to the vectors expressing VP₂ and VP₆ cDNAs.

Introns were also added to the vectors. Several introns of various origins were tested ; SV40 early genes, SV40 late genes, β-globin genes, EF1α gene, αs1-casein gene, rabbit WAP gene, bovine and human growth hormone genes.
Therefore, the invention encompasses the above described non-human mammal wherein the transgene further comprises one or several introns, such as but not limited to the above cited introns.

Enhancers were also added to the WAP gene promoter and to the transcribed region of the vectors: αs1-casein gene (in monomer or multimer), LTR from HTLV1 genome, immunoglobulin gene, LTR from MMTV genome, distal upstream region (up to 140 kb) and downstream region (at least 10 Kb) of the WAP genes (Rival-Gervier et al 2002 ; Rival et al EP 1 217 071, WO 0205203) ; β-globin gene. Therefore, the invention encompasses the above described non-human mammal wherein the transgene further comprises one or several enhancers located in the promoter region and/or in the transcribed region, such as but not limited to the above cited enhancers.

Several transcription terminators : from SV40 early and late genes, from β-globin genes, from WAP gene, from bovine and human growth hormone were used. As a result, the invention concerns the above described non-human mammal wherein the transgene further comprises one or several transcription terminators, such as but not limited to the above cited terminators.

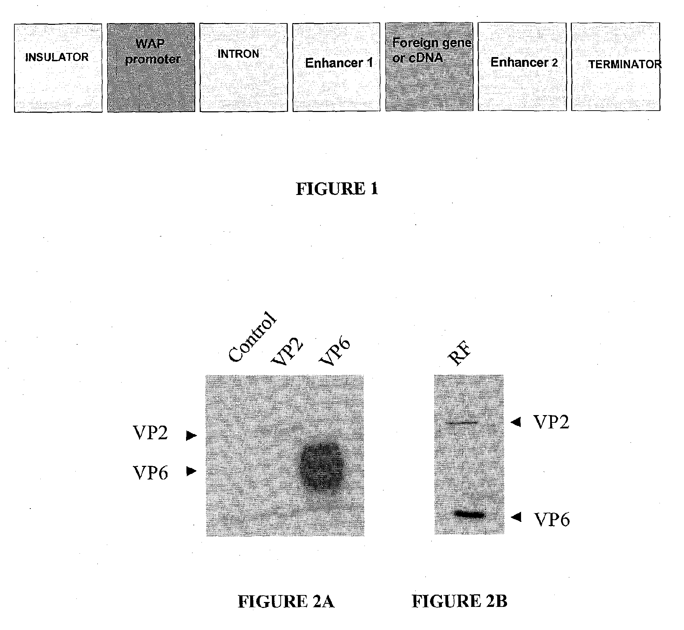

An example of common structure of the vectors comprising the above elements is shown in Fig 1.

In still another embodiment, the invention is directed to the above non-human transgenic mammal, wherein at least two cDNAs encoding a rotavirus protein selected from VP2, VP4, VP6 and VP7 are within one single said transgene.
cDNAs may be optimized for production in mammary tissue and secretion in milk. In this regards, cryptic splicing sites present in VP₂ and VP₆ cDNAs can be inactivated, several sequences potentially capable of altering transcription, translation or of reducing mRNA stability can be mutated as well as glycosylation sites. An optimization of some codons can also be done to further favor cDNA expression in mammalian cells.

In the context of the invention, the transgene may further comprise a coding sequence for an exogenous or endogenous peptide or protein or epitope thereof. This leads to non-human transgenic mammals producing recombinant VLP harboring epitopes in the milk.

The expression "mammal" is meant to include sheep, pig, goat, cow, rabbit, rat or mouse.

In a second aspect, the invention relates to a method for producing a recombinant rotavirus VLP or protein parts of VLP comprising the steps of:
(a) inserting into a non-human mammalian embryo or fertilized egg a transgene as defined above,
(b) allowing said embryo or fertilized egg to develop into an adult mammal,
(c) inducing lactation in said non-human mammal, or in a female descendant of said non-human mammal in which said transgene is present in the mammary tissue genome,
(d) collecting milk of said lactating non-human mammal, and
(e) isolating said VLP or protein parts of VLP from said collected milk.

In other words, the invention contemplates a method for producing a recombinant rotavirus VLP or protein parts of VLP comprising the steps of:
(a) inducing lactation in a transgenic non-human mammal as defined above, or in a female descendant of said non-human mammal,
(b) collecting milk of said lactating non-human mammal, and
(c) isolating said VLP or protein parts of VLP from said collected milk.

The expression "protein parts of VLP" refers to monomers, dimers, trimers or other homo or hetero multimers of proteins selected from VP2, VP4, VP6 and VP7. It also embraces proteins aggregates of VP2 and VP6.

In a preferred embodiment, VP2 and VP6 that are present in milk are not degraded, not cleaved and not glycosylated.

In the above method, said protein parts of VLP may be purified, eventually dissociated, and brought into contact in conditions to reassemble recombinant VLP. The purification step may comprise a first step consisting of preparing lactoserum.

In a third aspect, the invention is aimed at the use of a recombinant epitope harboring VLP obtained from the non human transgenic animal or the method as described above for the manufacture of an immunogenic composition, such as a vaccine, for treating or preventing infection with parasites, bacteria or virus, including but not limited to HIV, papilloma, herpes, hepatitis A, B or C, RSV, coronavirus, foot an mouth disease, rotavirus, Aujeszki disease, Marek disease.

It also concerns the use of a recombinant epitope harboring VLP obtained from the non human transgenic animal or the method as defined above for the manufacture of an immunogenic composition, such as a vaccine, for treating or preventing cancer, auto-immune diseases and metabolic disorders.

The invention is further embodied in the following examples and figures.

### Figure legends

**Figure 1**: Structure of the vectors used to produce rotavivus recombinant protein in milk. The different elements of the vectors are depicted herein in the text.
**Figure 2:** Secretion of VP2 and VP6 from transfected CHO cells.
   **(A)** CHO cells were tranfected with expression vectors containing wild type VP2 or VP6 cDNAs fused to a signal peptide. Transfected cells were grown for 48h and culture media were then collected, concentrated on Vivaspin concentration columns (Vivascience) and subjected to Western blot analysis with a polyclonal anti-rotavirus antibody (8148) followed by chemiluminescence detection (ECL, Amersham Bioscience). Culture media from cells transfected with VP2 or VP6-containing plasmid are noted VP2 and VP6 respectively. Control refers to non transfected cells. Positions of the different proteins are indicated by arrowheads. **(B)** Electrophoresis carried out with bovine rotavirus strain RF.

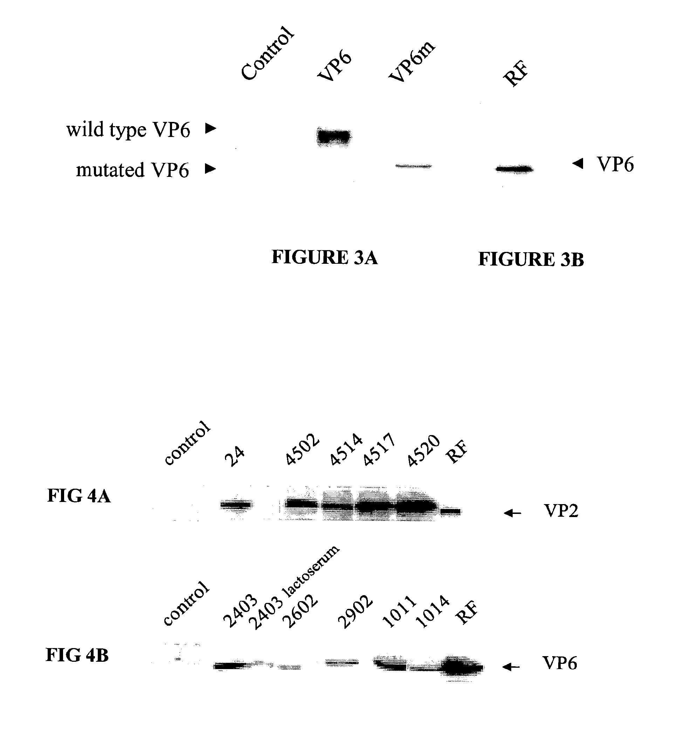

**Figure 3:** Comparison of the electrophoretic mobility of mutated versus wild type VP6. **(A)** The experiment was carried out as depicted in the legend of figure 2 with vectors containing wild type and mutated VP6 cDNAs fused to signal peptide (respectively VP6 and VP6m). **(B)** Position of wild type virus (RF) VP6 protein is shown and indicated by arrowhead.
**Figure 4:** Presence of VP2 and VP6 in milk of transgenic mice.
   Fractions of defatted milk or lactoserum from either non-transgenic animals (control milk), or from the transgenic lines 10, 24, 26, 29 and 45 were added in each lane for western blot analysis. The first two figures indicate the number of the lines. Lactoserum was the supernatant of defatted milk from which caseins have been precipitated by adding CaC12. The samples were diluted in Laemmli buffer, boiled for 5 minutes and loaded on a 12% polyacrylamide gel for SDS-PAGE. The proteins were then transferred on a PVDF membrane, blotted with **(A)** a monoclonal anti-VP2 antibody (E22) or **(B)** a polyclonal anti-rotavirus antibody (8148) and detected by chemiluminescence (ECL, Amersham Bioscience). The mouse 1014 contained less transgene copies than the mouse 1011 due to a segregation of the transgene integrated in several independent sites of the genome.This antibody recognizes much better VP6 than VP2.

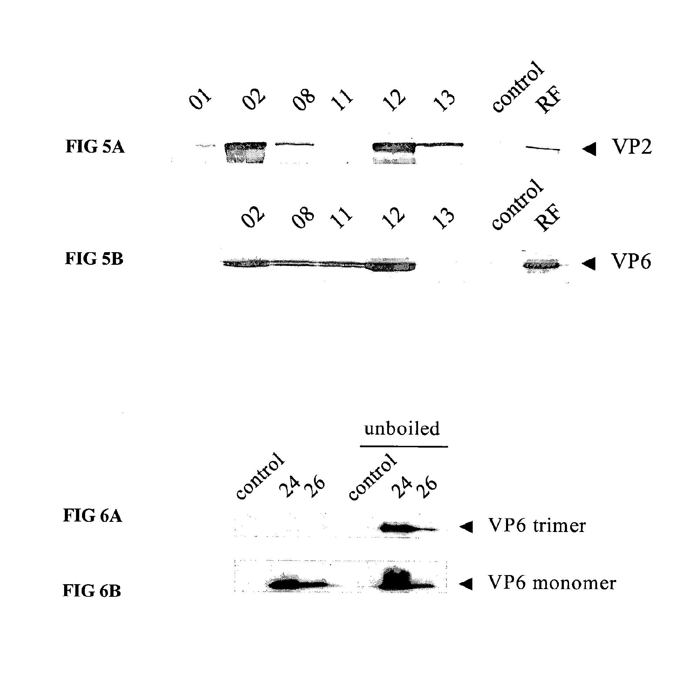

**Figure 5:** Presence of VP2 and VP6 in the lactoserum of transgenic rabbits.
   **(A)** VP2 and **(B)** VP6 in the lactoserum of transgenic rabbits lines 01, 02, 08, 11, 12, 13 were visualized by Western blot using (A) a monoclonal anti VP2 antibody (E22) or (B) a polyclonal anti-rotavirus antibody (8148).
**Figure 6:** Trimerisation of VP6 in the milk of transgenic F0 mice.
   Fractions of defatted milk from either non-transgenic animals (control), or transgenic mice lines 24 and 26 were loaded in each lane. Samples were either diluted in Laemmli buffer and boiled for 5 minutes or directly loaded (unboiled) on a 12% polyacrylamide gel for SDS-PAGE. Proteins were then transferred on a PVDF membrane, blotted with a polyclonal anti-rotavirus antibody and detected by chemiluminescence (ECL, Amersham Bioscience). The mouse 1014 contained less transgene copies than the mouse 1011 due to a segregation of the transgene integrated in several independent sites of the genome.

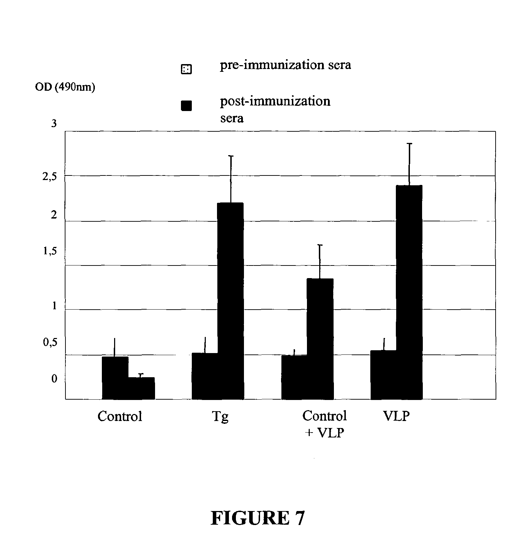

**Figure 7:** Serum IgG anti-VP2-VP6 antibodies after subcutaneous immunization with transgenic milk.
   IgG anti-VLP 2/6 antibodies were measured in mice after two subcutaneous inoculations. Sera were collected 14 days post inoculation. The volume of milk corresponding to 1.5µg of VP2 and VP6 were injected into mice (Tg). Control immunizations were carried out using control milk (control), 1µg of pure VLP 2/6 synthesized by baculovirus **(VLP),** and 1µg of VLP 2/6 added in control milk (control + **VLP).** Antibodies were detected in individual mice by ELISA. Data are the means of IgG levels ± SEM at a serum dilution of 1/900.

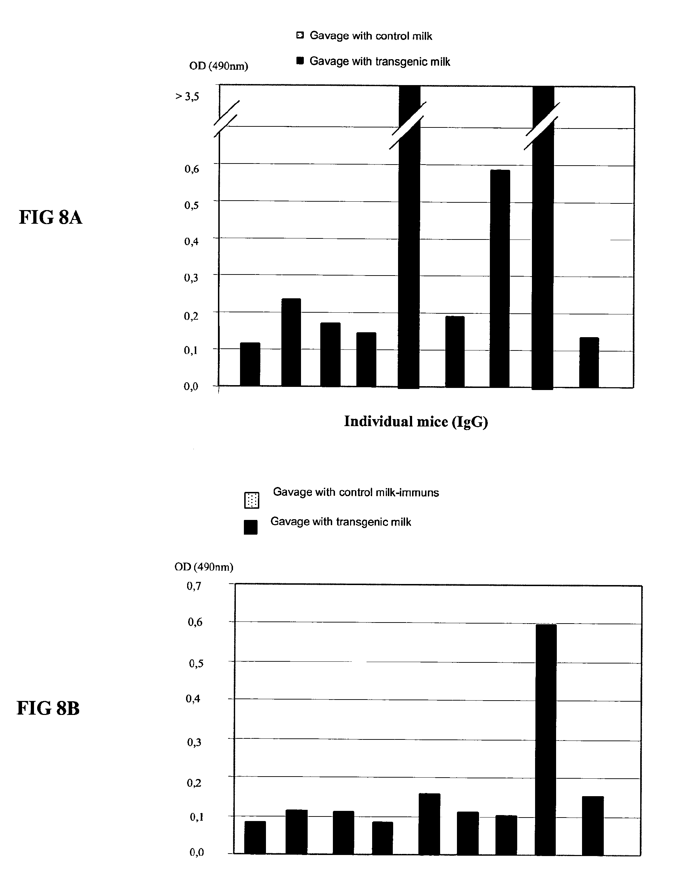

**Figure 8:** Serum IgG and fecal IgA anti-VP2-VP6 antibodies after oral immunization with transgenic milk.
   **(A)** IgG anti VLP 2/6 antibodies were measured in mice after three oral administrations of milk from transgenic or control rabbits. Sera were collected 14 days after the last gavage. The volume of milk corresponding to about 35 µg of each protein VP2 and VP6 was administered to each mouse. Control immunizations were carried out using same volume of milk from non-transgenic animals. Antibodies were detected in individual mice by ELISA. Data correspond to IgG levels at a serum dilution of 1/900. **(B)** IgA levels in stool samples measured in the same animals (1/5 dilution).

### Example 1: Modifications of VP₂ and VP₆ cDNAs

Rotavirus genome is formed by several RNA fragments which are replicated and expressed in the cytoplasm of infected cells. These sequences have therefore no intron but may contain cryptic splicing signals. The rotavirus RNAs do not contain signal for the transfer from the nucleus to the cytoplasm. On the other hand, the viral proteins are synthesized, assembled in cytosol and not secreted by exocytosis after having transited through endoplasmic reticulum and Golgi apparatus.

A high number of nucleotide sequence modifications have been done in the two cDNAs leading to only a few alterations of amino acid sequences. Signal peptides from different mammalian proteins (bovine or human growth hormone family, milk proteins ...) have been added to naturally non-secreted proteins, allowing an efficient secretion of these proteins. Several of these signal peptides were compared to allow further optimization.

Some of the cryptic splicing sites present in VP₂ and VP₆ cDNAs were inactivated. Several sequences potentially capable of altering transcription, translation or of reducing mRNA stability were mutated. An optimization of some codons was also done to favor cDNA expression in mammalian cells.

The expression of wild type and optimized cDNAs was compared in transfected CHO cells using several vectors (pcDNA3 (Invitrogen ,VT90-20), pEF0 (Taboit-Dameron et al, 1999). The presence of the proteins VP₂ and VP₆ in culture medium was revealed using Western blot analysis.

Data shown in Figure 2 indicate that both VP₂ and VP₆ were secreted from transfected CHO. This demonstrates, for the first time, that rotaviral proteins can be secreted at a high rate from animal cells after the addition of a signal peptide. This indicates that, if signals targeting proteins to some cytosol compartments are present in VP₂ and VP₆, their effect is of limited importance when a signal peptide is added to them. Wild typeVP6 was expressed as a protein having a higher molecular weight than the viral protein. This may be due to a glycosylation of VP6 which does not occur in the viral protein but may take place when the protein migrates through endoplasmic reticulum and Golgi apparatus. The mutated VP6 devoid of most of its glycosylation sites migrated at the same level as the viral protein (Figure 3).The presence of carbohydrates in VP6 might alter VLP formation or reduce its immunological properties.

### Example 2: Vectors for specific secretion in milk

The above elements in example generated are combined to form a broad family of vectors which were tested in CHO cells or in mouse mammary HC11 cells as well as rabbit primary mammary cells. Although poorly predictive of the expression levels in transgenic animals, the cellular tests made it possible the elimination of the combinations which showed the lowest potency. The different vectors containing optimized/mutated compared to wild type VP₂ and VP₆ cDNAs allowed an increase of expression of 10, 000 fold. Ultimately, our work on the optimization of the vector led to non-human transgenic mammals producing of 100 µg/ml of both proteins in milk.

### Example 3: Characterization of the proteins VP₂ and VP₆ from milk

Western blot analysis revealed that the optimized concentration of VP₂ and VP₆ in milk was 100 µg/ml or more according to the lines of transgenic animals (Figures 4 and 5 and Table 1).

**Table 1:**

| **Measurement of VP2 and VP6 in the milk of transgenic mice.** | | | | |
|---|---|---|---|---|
| Transgenic mouse lines | VP2 Tg | VP6 Tg | VP2 in milk (µg/ml) | VP6 in milk (µg/ml) |
| 03 | + | + | 0 | 10-20 |
| 05 | + | + | 100 | 100 |
| 10 | + | + | 0 | 10-50 |
| 24 | + | + | 50-100 | 100 |
| 26 | + | + | 0 | 25 |
| 29 | + | + | 0 | 30 |
| 45 | + | - | 80-100 | 0 |

The concentration of the recombinant proteins was determined by Western blot assays using the viral proteins as a reference. VP2 Tg : transgenic for VP2, VP6 Tg : transgenic for VP6.

**Table 2:**

| **Measurement of VP2 and VP6 in the milk of transgenic rabbits.** | | | | |
|---|---|---|---|---|
| Transgenic rabbit lines | VP2 Tg | VP6 Tg | VP2 in milk (µg/ml) | VP6 in milk (µg/ml) |
| 01 | + | - | 20-30 | 0 |
| 02 | + | + | 80-100 | 70 |
| 08 | + | + | 30 | 60 |
| 11 | + | + | 0 | 50 |
| 12 | + | + | 100 | 250 |
| 13 | + | - | 30-50 | 0 |

The concentration of the recombinant proteins was determined by Western blot assays using the viral proteins as a reference. VP2 Tg : transgenic for VP2, VP6 Tg : transgenic for VP6.

The two proteins were at the expected molecular weight after complete denaturation in Western blot assays (Figures 4 and 5). This indicates that the proteins were not cleaved, degraded or glycosylated. Interestingly, VP₆ was in form of trimer when denaturation did not include the heating step (figure 5). This property of VP₆ was found with the nascent and the recombinant protein extracted from Sf9 cells infected by a baculovirus harboring the VP₆ cDNA.
VP₂ and VP₆ were identified by Western blot assays in lactoserum (obtained after a specific precipitation of caseins by adding an excess of calcium) (Figures 4 and 5). This indicates that the proteins VP₂ and VP₆ were not aggregated to casein micelles and that preparation of lactoserum is likely an efficient first step for the purification of the viral proteins.

An ultracentrifugation of the lactoserum pelleted both VP₂ and VP₆. VP₂ which is known to form core-like aggregates was pelleted alone but not VP₆ which was found in the pellet only in the presence of VP₂. This strongly suggests that recombinant VP₂ and VP₆ spontaneously form VLP in milk as in other systems such as Sf9- baculovirus.
A centrifugation in CsCl gradients is acknowledged to concentrate rotavirus, nascent VLP and recombinant VLP prepared by the baculovirus-Sf9 cells system. The floating material in CsCl gradients contains capsids having the morphological characteristics of rotavirus under electron microscopy observation.

Lactoserum from transgenic animals was fractionated in Superose12.VP2 and VP6 were coeluted and exclusively in the void volume. This indicates that the two proteins are associated forming aggregates of at least 300,000 KDa. The material found in the void volume of was subjected to CsCl gradient ultracentrigation. The VP2 and VP6 proteins were found in the gradient at the density corresponding to native VLP.
The exact structure of the VP2-VP6 aggregate found in milk might form an incomplete capsid. Indeed, VP2 and VP6 were at similar concentrations in milk whereas VP6 is approximately 6 times more abundant than VP2 in the virus. Nevertheless, VLP can be prepared from the VP2/VP6 milk extracted and purified proteins, adjusting the ratio for in vitro re-assembly. It will also be understood that the production ratio in milk may be modulated using different sets or number of vectors expressing VP2 and VP6.

### Example 4: Immunization of mice with milk containing VP2 and VP6

Defatted rabbit milk (30µl) was administered to mice by subcutaneous injections in the presence of incomplete Freund adjuvant. Two weeks later the treatment was repeated. Alternatively, defatted milk (500µl) mixed with cholera toxin (5µg) was orally administered to mice (3 times with one week interval between gavages). One week after the last injection or gavage, blood and stool samples were collected from the animals and the presence of anti-VP2 and anti-VP6 antibodies was searched.
High amounts of anti-VP6 IgG antibodies were found in the serum of the seven immunized mice. Only a background of natural antibodies binding to VP6 was present in the serum of control mice which received milk from non transgenic animals (Figure 7). Quite significant amounts of anti-VP6 IgG antibodies were also found in the serum of three out of five mice which received orally 500µl of milk from transgenic rabbits of line 02. This volume of milk contained about 35µg of each protein VP2 and VP6.
Control mice having received the same volume of milk from non-transgenic rabbits had only a background of VP6 binding proteins (Figure 8A).
Interestingly, Anti-VP6 IgA were found in stool samples of three of the mice immunized by gavage (Figure 8B). The response to the antigens was very weak in two mice. This may be due to the fact that relatively small quantities of the viral proteins were administered orally.

These data report for the first time that proteins VP₂ and VP₆ from rotavirus can be secreted and co-secreted in cultured cells and *in vivo,* in milk. The amount of secreted proteins is much higher than this obtained with other systems. This offers the possibility to produce new low cost and safe vaccines. Protocols involving the administration of relatively high amounts of proteins without any adjuvant may now be much more easily implemented.

The proteins VP₂ and VP₆ form a VLP which can be used as vaccine administered by injection or orally.

The production system described here is appropriate to express other rotavirus proteins such as VP₄ and VP₇ which may also be used as vaccines, alone or associated to VP₂ and VP₆.

The addition of foreign peptides or proteins to VP₂ and VP₆ does not prevent the formation of VLP and allow the generation of antibodies against the foreign epitopes. The method described here allows the preparation of recombinant VLP harboring epitopes from other human or animal virus such as HIV, papilloma, herpes, hepatitis A, B or C, RSV, coronavirus, foot an mouth disease, Aujeszki disease, Marek disease ... or from pathogenic bacteria and parasites. This vaccination approach through the oral route may be particularly important when the production of IgA is required to eradicate a pathogen.

Another therapeutic application is to use recombinant rotavirus VLP as carrier for epitopes of endogenous genes, to induce immune response and reduce action of molecules such as those involved in cancer, autoimmune diseases, metabolic disorders.

### REFERENCES

Acharya T., Daar A.S.,Singer P.S.(2003)Biotechnology and the LTN's millenium development goals.Nature Biotechnol. 21:1434-1436
Beale, B. (2002). Rotavirus vaccines, take two. *Scientist* August 19 : 34-35.
Bertolotti-Ciarlet A.,Ciarlet M.,Crawford S.E.,Conner M.E. and Estes M.K.(2003) Immunogenicity and protective efficacy of rotavirus2/6 virus-like particles produced by dual baculovirus expression vector and administered intramuscularly, intranasally, or orally to mice.Vaccine 21:3885-3900.
Ciarlet M., Crawford S.E., Barone C., Bertelotti-Cialet A., Ramig M.F., Estes M.K., Conner M.E. (1998) Subunit rotavirus vaccine administered parenterally to rabbits induces active protective immunity.J.Virol. 72:9233-9246.
Charpilienne, A., Nejmeddine, M., Berois, M., Parez, N., Neumann, E., Hewat, E., Trugnan, G., and Cohen, J. (2001). Individual rotavirus-like particles containing 120 molecules of fluorescent protein are visible in living cells. *J. Biological* 276 : 29361-29367.
Kim Y., Nielsen P.R., Hodgins D, Chang K.O., Saif I.J. (2002) Lactogenic antibody responss in cows vaccinated with recombinant bovine rotavirus-like particles (VLPs) of two seotypes or inactivated bovine rotavirus vaccines.Vaccine.20:1248-1258.
Jiang B., Estes M.K., Barone C., Barniak V., O'Neal C.M., Ottaiano A., Madore H.P. and Conner M.E. Vaccine 17:1005-1013.
Ma, J.K-C, Drake, P. M.W., and Christou, P., (2003). The production of recombinant pharmaceutical proteins in plants. *Nature Review* 4 : 794-805.
Matsumura, T., Itchoda, N., and Tsunemitsu, H. (2002). Production of immunogenic VP6 protein of bovine group A rotavirus in transgenic potato plants. *Arch. Virol.* 147 /1263-1270.
O'Neal C.M., Crawford S.E., Estes M.K., Conner M.E. (1997), Rotavirus- like particles administered mucosally induce protective immunity. J.Virol. 71:8707-8717.
Petitclerc, D., Attal, J., Theron, M.C., Bearzotti, M., Bolifraud, P., Kann, G., Stinnakre, M.G., Pointu, H., Puissant, C., and Houdebine, L.M. (1995). The effect of various introns and transcription terminators on the efficiency of expression vectors in various cultured cell lines and in the mammary gland of transgenic mice. *J*. *Biotechnol.* 40 : 169-178.
Rival S., Viglietta C., Attal J., Houdebine L.M. (2002) Position-independent and tissue-specific expression of a transgene in milk of transgenic animals; EP 1 217 071 and WO 0205203.
Rival-Gervier S., Pantano, T., Viglietta, C., Maeder, C., Prince, S., Attal, J., Jolivet, G., and Houdebine, L.M. (2003). The insulator effect of the 5'HS4 region from the β-globin chicken locus on the rabbit WAP gene promoter activity in transgenic mice. *Transgenic Res*.12:723-730
Rival-Gervier S., Viglietta C., Maeder C., Attal J., Houdebine L.M.(2002) Position-independent and tissue-specific expression of porcine whey acidic protein gene from a bacterial artificial chromosome in transgenic mice.Mol.Reprod. Develop. 63:161-167
Schwartz-Cornil I., Benureau Y., Greeberg H., Hendrickson B.A., Cohen J.(2002) Heterologous protection induced by the inner capsid proteins of rotavirus requires transcytosis of mucosal immunoglobulins.J.Virol. 76:8110-8117
Taboit-Dameron, F., Malassagne, B., Viglietta, C., Puissant, C., Leroux-Coyau, M., Chereau, C., Attal, J., Weill, B., and Houdebine, L.M. (1999). Association of the 5'HS4 sequence of the chicken beta-globin locus control region with human EF1 alpha gène promoter induces ubiquitous and high expression of human CD55 and CD59 cDNAs in transgenic rabbits. *Transgenic Res.* 8 : 223-235.
Wu, Y.Z., Li, J.T., Mou, Z.R., Fei, L., Ni, B., Geng, M., Jia, Z.C., Zhou, W., Zou L.Y., and Tang Y. (2003). Oral immunization with rotavirus VP7 expressed in transgenic potatoes induced high titers of mucosal neutralizing IgA. *Virol.* 313 : 337-342.
Yu, J., and Langridge, W. (2003). Expression of rotavirus capsid protein VP6 in transgenic potato and its oral immunogenicity in mice. *Transgenic Res.* 12 : 163-169.

## Claims

1. A non-human transgenic mammal whose genome comprises:
i) a first transgene comprising a mammary gland specific transcriptional control region operably linked to cDNA encoding a rotavirus protein selected from VP2, VP4, VP6 and VP7 and wherein said cDNA comprises a secretion signal sequence;
ii) at least a second transgene comprising a mammary gland specific transcriptional control region operably linked to cDNA encoding another rotavirus protein selected from VP2, VP4, VP6 and VP7 and wherein said cDNA comprises a secretion signal sequence; and wherein said rotavirus protein are secreted separately and auto-assembled in milk in rotavirus like particles (VLP) or aggregates of said rotavirus proteins.

2. The non-human transgenic mammal according to claim 1, wherein the first transgene comprises a cDNA encoding a VP2 rotavirus protein and the second transgene comprises a cDNA encoding a VP6 rotavirus protein.

3. The non-human transgenic mammal according to claim 2, further comprising a third or fourth transgene comprising a cDNA encoding a rotavirus protein selected from VP4 and VP7.

4. The non-human transgenic mammal according to one of claims 1 to 3, wherein said VP2 and VP6 assemble in VLP of at least 300 000 KDA.

5. The non-human transgenic mammal according to one of claims 1 to 4, wherein the milk contains at least 10 µg/ml, preferably at least 100 µg/ml of both VP2 and VP6.

6. The non-human transgenic mammal according to one of claims 1 to 5, wherein said mammary gland specific transcriptional control region is selected from a milk serum protein or a casein protein, in particular the WAP promoter such as the long mouse or rabbit WAP promoter.

7. The non-human transgenic mammal according to claim 6, wherein said mammary gland specific transcriptional control region is the long WAP rabbit promoter, such as a region of at least 3 kb, 3 kb to 6.3 kb or at least 6.3 kb from the translation initiation start of the rabbit WAP promoter.

8. The non-human transgenic mammal according to one of claims 1 to 7, wherein the transgene further comprises the genomic sequences surrounding the WAP gene, preferably at least 110Kb upstream and at least 10Kb downstream of the WAP gene from sheep, pig, goat, cow, rabbit, rat or mouse.

9. The non-human transgenic mammal according to one of claims 1 to 8, wherein the transgene further comprises the 5'HS4 region from the chicken β-globin gene cluster.

10. The non-human transgenic mammal according to one of claims 1 to 9, wherein the transgene further comprises one or several introns, such as introns of SV40 early genes, SV40 late genes, β-globin genes, EF 1α gene, αs1-casein gene, rabbit WAP gene, bovine and human growth hormone genes.

11. The non-human transgenic mammal according to one of claims 1 to 10, wherein the transgene further comprises one or several enhancers located in the promoter region and/or in the transcribed region, such as enhancers of the αs1-casein gene (in monomer or multimer), LTR from HTLV1 genome, immunoglobulin gene, LTR from MMTV genome, distal upstream regions (up to 140 kb) of the WAP genes and β-globin gene.

12. The non-human transgenic mammal according to one of claims 1 to 11, wherein the transgene further comprises one or several transcription terminators, such as terminators of the SV40 early and late genes, β-globin genes, WAP gene, and bovine and human growth hormone.

13. The non-human transgenic mammal according to one of claims 1 to 12, wherein at least two cDNAs encoding a rotavirus protein selected from VP2, VP4, VP6 and VP7 are within one single said transgene.

14. The non-human transgenic mammal according to one of claims 1 to 13, wherein the transgene further comprises a coding sequence for an exogenous or endogenous peptide or protein or epitope thereof and wherein non-human transgenic mammal produce recombinant VLP harboring epitopes in the milk.

15. The non-human transgenic mammal according to one of claims 1 to 14, said mammal being a sheep, pig, goat, cow, rabbit, rat or mouse.

16. A method for producing a recombinant rotavirus VLP or protein parts of VLP comprising the steps of:
(a) inserting into a non-human mammalian embryo or fertilized egg a transgene as defined in one of claims 1 to 15,
(b) allowing said embryo or fertilized egg to develop into an adult mammal,
(c) inducing lactation in said non-human mammal, or in a female descendant of said non-human mammal in which said transgene is present in the mammary tissue genome,
(d) collecting milk of said lactating non-human mammal, and
(e) isolating said VLP or protein parts of VLP from said collected milk.

17. A method for producing a recombinant rotavirus VLP or protein parts of VLP comprising the steps of:
(a) inducing lactation in a transgenic non-human mammal according to one of claims 1 to 15, or in a female descendant of said non-human mammal,
(b) collecting milk of said lactating non-human mammal, and
(c) isolating said VLP or protein parts of VLP from said collected milk.

18. The method according to claim 17, wherein protein parts of VLP comprise trimers of VP6.

19. The method according to claim 17, wherein VP2 and VP6 present in milk are not degraded, not cleaved and not glycosylated.

20. The method according to claim 16 to 19, wherein said protein parts of VLP are purified, eventually dissociated, and brought into contact in conditions to reassemble recombinant VLP.

21. The method according to claim 16 to 20, wherein the purification comprises a first step consisting of preparing lactoserum.

22. The use of a recombinant epitope harboring VLP obtained from a non human transgenic animal according to one of claims 1 to 15 or the method as defined in one of claims 16 to 21 for the manufacture of an immunogenic composition, such as a vaccine, for treating or preventing infection with parasites, bacteria or virus, including HIV, papilloma, herpes, hepatitis A, B or C, RSV, coronavirus, foot an mouth disease, rotavirus, Aujeszki disease, Marek disease.

23. The use of a recombinant epitope harboring VLP obtained from a non human transgenic animal according to one of claims 1 to 15 or the method as defined in one of claims 16 to 21 for the manufacture of an immunogenic composition, such as a vaccine, for treating or preventing cancer, auto-immune diseases and metabolic disorders.
